# EUROPEAN PATENT SPECIFICATION

(11) **EP 2 613 686 B1**
(45) Date of publication and mention of the grant of the patent: **19.09.2018**
(21) Application number: 11842688.1
(22) Date of filing: 02.08.2011
(51) Int. Cl.: A61B 5/042, A61B 5/00, A61M 25/00, A61B 18/14, A61M 25/06, A61M 25/01, A61B 18/00

(54) **MEDICAL DEVICES HAVING AN ELECTROANATOMICAL SYSTEM IMAGING ELEMENT MOUNTED THEREON**
MEDIZINISCHE VORRICHTUNG MIT EINEM DARAUF MONTIERTEN BILDGEBUNGSELEMENT EINES ELEKTROANATOMISCHEN SYSTEMS
DISPOSITIFS MÉDICAUX SUR LESQUELS EST MONTÉ UN ÉLÉMENT D'IMAGERIE D'UN SYSTÈME ÉLECTROANATOMIQUE

(30) Priority: 23.11.2010 US 952948; 30.12.2010 US 982675
(43) Date of publication of application: 17.07.2013
(73) Proprietor: St. Jude Medical Atrial Fibrillation Division Inc., St. Paul, Minnesota 55117-9913 (US)
(72) Inventor: KAUPHUSMAN, James V., Champlin, Minnesota 55316 (US); GRASSE, Martin M., Boston, Massachusetts 02116 (US); FUENTES, Allan, Mound, Minnesota 55364 (US); GONZALEZ, Salome A., Maple Grove, Minnesota 55369 (US)
(74) Representative: Kramer Barske Schmidtchen Patentanwälte PartG mbB
(86) International application number: PCT/US2011/046266
(87) International publication number: WO 2012/071087

(56) References cited:
- EP-A2- 0 226 397
- US-A- 5 662 606
- US-A- 5 772 642
- US-A1- 2002 072 710
- US-A1- 2003 130 598
- US-A1- 2008 091 169
- US-A1- 2008 139 999
- US-A1- 2009 228 092
- US-A1- 2009 262 979
- US-B1- 6 379 346
- US-B1- 7 826 881
- US-B2- 7 468 027
- US-B2- 7 699 771

## Description

### BACKGROUND OF THE INVENTION

### a. Field of the Invention

This disclosure relates to a family of medical devices. More particularly, this disclosure relates to medical devices, such as, for example, deflectable catheter-introducers or sheaths, having one or more electrodes mounted thereon for electrophysiology (EP) diagnostics and localization and visualization of said devices, as well as methods of manufacturing and systems with which such medical devices are used, including robotic surgical systems.

### b. Background Art

It is well known to use a medical device called a sheath or catheter-introducer when performing various therapeutic and/or diagnostic medical procedures on or in the heart, for example. Once inserted into a patient's body, these particular medical devices (hereinafter referred to as "sheaths") provide a path through a patient's vasculature to a desired anatomical structure or site for a second medical device, such as, for example, a catheter, a needle, a dilator, etc., and also allow for the proper positioning or placement of the second medical device relative to the desired anatomical structure.

One drawback to conventional sheaths and their use is that visualization of the sheath and/or its position has proved difficult, if not impossible. As a result, physicians have been unable to see the sheath and/or its position during the performance of a medical procedure without the use of ionizing radiation (*e.g*., acute x-ray delivery via a fluoroscope). However, with the advent and growing use of various automated guidance systems, such as, for example, magnetic-based and robotic-based guidance systems, the need for such visualization capability has increased. More particularly, it is important for the physician/clinician operating such automated systems to know and understand exactly where the various medical devices being used are located and how they are oriented.

In addition to the need of visualization in the use of automated guidance systems, the need for this capability is also increasing in instances where a physician manually controls medical devices. For example, for procedures performed on the left side of the heart, a transseptal puncture is used to cross the septum separating the right atrium from the left atrium. In such procedures, a long, small diameter needle is passed down a lumen in the sheath and is used to puncture the septal wall. Once formed, the sheath is inserted into the hole created by the puncture operation and crosses through the septum, thereby providing another medical device within the sheath access to the left atrium. Using current visualization systems, such as, for example, fluoroscopy, the transseptal crossing point (and the sheath therein) is invisible to the physician. Accordingly, if the physician loses visual contact with a device or the transseptal access is interrupted due to, for example, patient movement or the manipulation of a medical device used with the sheath, regaining access increases the procedure time and also can require another puncture of the septum. Because there is no visualization of the sheath, or any representation of the sheath on a display the physician is using, the physician has no reference to help guide him to regain access.

US 6,379,346 B1 discloses an introducer comprising an elongated tube having first and second lumens, a molded handle coupled to the proximal end of the tube, having a proximal opening aligned with the first lumen of the tube and having a wall adjacent the opening of the handle which defines a slot, located diametrically opposite the second lumen of the tube, and a tubular reinforcement member located within the second lumen of the tube, the handle also being provided with a second opening, open to the second lumen of the tube.

US 2009/0262979 A1 discloses a system for determining a flow of material in a volume of a heart of a patient.

US 2003/0130598 A1 discloses a guide catheter, comprising an outer sheath comprising an open lumen and a pre-shaped distal end, an inner sheath comprising an open lumen, the inner sheath disposed within the open lumen of the outer sheath, the inner sheath axially rotatable and longitudinally translatable relative to the outer sheath, a distal end of the inner sheath conforming to a shape of the outer sheath when the inner sheath is retracted, and the distal end of the inner sheath assuming a pre-formed shape when the distal end of the inner sheath is extended beyond the distal end of the outer sheath, a steering tendon disposed along the outer sheath, a distal end of the steering tendon connected to a distal tip of the outer sheath, a guide handle connected to a proximal end of the outer sheath, and a steering mechanism disposed on the guide handle, the steering mechanism connected to a proximal end of the steering tendon and providing a pulling force on the steering tendon to adjustably change a shape of the pre-shaped distal end of the outer sheath.

US 2008/0091169 A1 discloses a catheter assembly comprising an inner liner made of flexible material and an outer layer having a steering mechanism, the steering mechanism comprising at least one flat wire and a corresponding lumen for each of the at least one flat wire, through which the flat wire may travel, wherein the outer layer comprises a braided wire assembly that includes at least two flat wires braided into a wire mesh.

US 0 226 397 A2 discloses an introducer for a catheter comprising a tubular member having a distal arm extending axially thereof, and a tube attached to said arm and extending proximally into said tubular member and being spaced from the inner wall thereof. A catheter adapted to perform electrophysiological procedures is known from US 5,662,606 A. US 2002/0072710 A1 discloses a delivery catheter for delivering intra-bodily medical devices to an internal body site.

Accordingly, the inventors herein have recognized a need for sheath designs and methods of manufacturing that minimize and/or eliminate one or more of the deficiencies in conventional cardiac catheter-introducers and sheaths.

### BRIEF SUMMARY OF THE INVENTION

The present disclosure is directed to a family of medical devices, such as deflectable cardiac catheter-introducers and sheaths. Specifically, the present disclosure is directed to a medical device as claimed in claim 1 and a system as claimed in claim 8. Further developments of the present invention are set out in the dependent claims.

Aspects, features, details, utilities, and advantages of the present disclosure will be apparent from reading the following description and claims, and from reviewing the accompanying drawings.

### BRIEF DESCRIPTION OF THE DRAWINGS

Fig. 1 is a perspective view of an exemplary embodiment of a medical device in accordance with present teachings.
Figs. 2 and 3 are cross section views of the medical device illustrated in Fig. 1 taken along the lines 2/3-2/3 showing the shaft of the medical device in various stages of assembly.

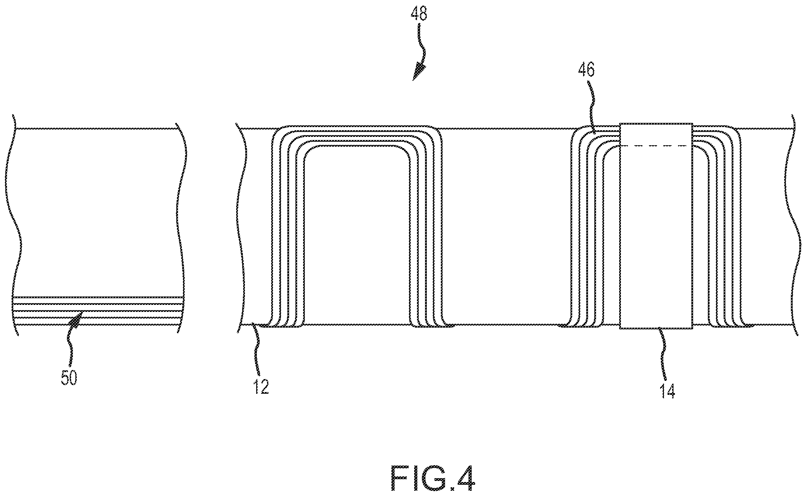

Fig. 4 is side view of a portion of an exemplary embodiment of the medical device illustrated in Fig. 1.
Fig. 5 is a cut-away perspective view of a portion of the medical device illustrated in Fig. 1.
Fig. 6 is a diagrammatic and schematic view of another exemplary embodiment of the medical device illustrated in Fig. 1 showing the medical device used in connection with an exemplary embodiment of an automated guidance system.
Fig. 7 is a diagrammatic and schematic view of the medical device illustrated in Fig. 5, wherein the distal end of the medical device is deflected.
Fig. 8 is a flow diagram illustrating an exemplary embodiment of a method of manufacturing a medical device in accordance with present teachings.
Fig. 9 is a diagrammatic view of a system for performing at least one of a diagnostic and a therapeutic medical procedure.
Fig. 10 is a simplified diagrammatic and schematic view of the visualization, navigation, and/or mapping system of the system illustrated in Fig. 9.
Fig. 11 is an exemplary embodiment of a display device of the system illustrated in Fig. 8 with a graphical user interface (GUI) displayed thereon.

### DETAILED DESCRIPTION OF EMBODIMENTS OF THE INVENTION

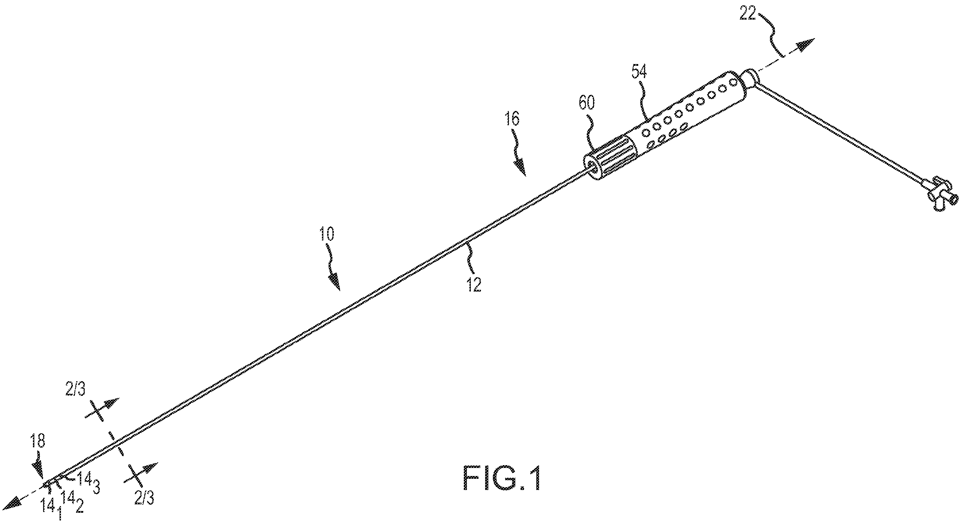

Referring now to the drawings wherein like reference numerals are used to identify identical components in the various views, Fig. 1 illustrates one exemplary embodiment of a medical device 10, such as, for example and without limitation, a sheath or cather-introducer for use in connection with a number of diagnostic and therapeutic procedures performed, for example, within the heart of a human being or an animal. For clarity and brevity purposes, the description below will be directed solely to a medical device 10 that comprises a sheath (sheath 10) for use in cardiac applications. It will be appreciated by those having ordinary skill in the art, however, that the description below can be applicable to medical devices other than sheaths, and for sheaths and medical devices used in connection with applications other than cardiac applications. Accordingly, medical devices other than sheaths, and medical devices/sheaths for use in applications other than cardiac applications, remain within the scope of the present disclosure.

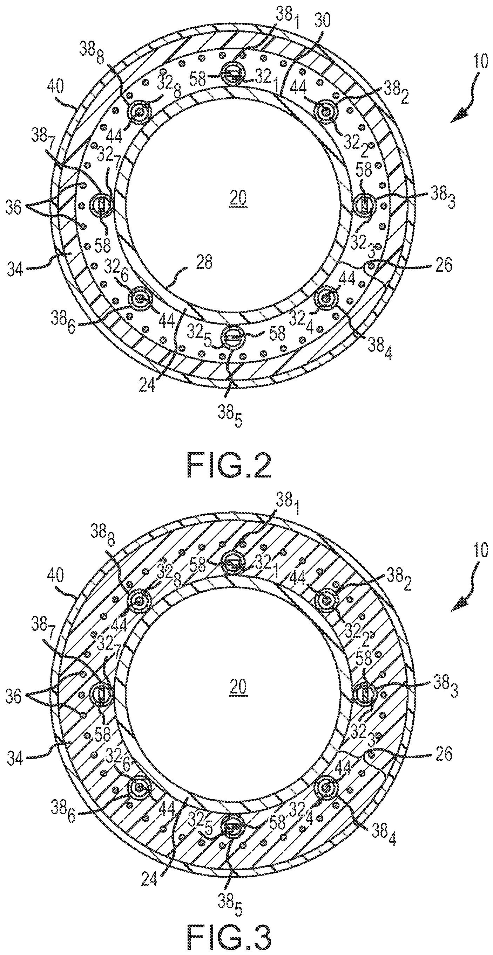

With reference to Fig. 1, in an exemplary embodiment, the sheath 10 comprises an elongate tubular shaft 12 and one or more electrodes 14 (*e.g*., 14₁, 14₂, 14₃ in Fig. 1) mounted thereon. The shaft 12 has a proximal end 16, a distal end 18, and a major lumen 20 (best shown in Figs. 2 and 3) extending between proximal and distal ends 16, 18 (as used herein, "proximal" refers to a direction toward the end of the sheath 10 near the physician/clinician, and "distal" refers to a direction away from the physician/clinician). The major lumen 20 defines a longitudinal axis 22 of the sheath 10, and is sized to receive a medical device therein. As illustrated in Fig. 1, and as will be described in greater detail below, the electrodes 14 are mounted on the shaft 12 at the distal end 18 thereof. However, in another exemplary embodiment, one or more of the electrodes 14 can be mounted at a location on the shaft 12 more proximal than the distal end 18. Additionally, the shaft 12 can have straight configuration, or alternatively, can have a fixed curve shape/configuration. The shaft 12 is configured for insertion into a blood vessel or another anatomic structure.

Figs. 2 and 3 are cross-section views of an exemplary embodiment of the shaft 12, wherein Fig. 2 illustrates the shaft 12 at a non-final stage of assembly, and Fig. 3 illustrates the shaft 12 at a final stage of assembly following the performance of a reflow process on at least a portion of the shaft 12. In this embodiment, and in its most general form, the shaft 12 comprises an inner liner 24 and an outer layer 26.

The inner liner 24 has an inner surface 28 and an outer surface 30, wherein the inner surface 28 defines the major lumen 20. In an exemplary embodiment, the inner liner 24 is formed of extruded polytretrafluoroethylene (PTFE) tubing, such as, for example, Teflon® tubing. In one exemplary embodiment, the PTFE comprises etched PTFE. An inner liner formed of this particular material creates a lubricious lumen (lumen 20) within which other medical devices used with the sheath 10, such as, for example, catheters, needles, dilators, and the like, can be passed. The inner liner 24 is relatively thin. For example, in one embodiment, the inner liner 24 has a thickness on the order 0.0015 inches (0.0381 mm). It will be appreciated by those having ordinary skill in the art that the inner liner 24 can be formed of a material other than PTFE, or etched PTFE. For example, in other exemplary embodiments, the inner layer 24 is comprised of polymeric materials, such as, for example and without limitation, polyether block amides, nylon, and other thermoplastic elastomers. Accordingly, sheaths having inner liners made of materials other than PTFE remain within the scope of the present disclosure.

With continued reference to Figs. 2 and 3, the outer layer 26 is disposed adjacent to the inner layer 24, and the outer surface 30 thereof, in particular. The outer layer 26 includes minor lumens 32 (*i.e.,* lumens 32₁-32₈ in Figs. 2 and 3) therein and coupled thereto adapted to receive and house, as will be described in greater detail below, deflectable elements, such as, for example, steering or pull wires associated with a steering mechanism for the sheath 10, or elongate conductors (*e.g.,* electrical wires) coupled to the electrodes 14. Because the major lumen 20 of the shaft 12 must be kept open to allow for the uninhibited passage of other medical devices therethrough, the minor lumens 32 are disposed within the outer layer 26 of the shaft 12.

The outer layer 26 can be formed of a single polymeric material, or alternatively, a combination of different components/materials (*e.g*., various tubing and braid assemblies) that, after the application of a reflow process on at least a portion of the shaft 12, combine to form the outer layer 26. In the exemplary embodiment illustrated in Fig. 2, the outer layer 26 comprises one or more layers of polymeric material that are placed over the inner liner 24. The polymeric material can be in the form of one or more extruded polymer tube(s) 34 sized so as to fit over the inner layer 24. The polymer tube 34 can comprise one or more of any number of polymeric materials, such as, for example and without limitation, polyether block amides (*e.g.,* Pebax®), polyamides (*e.g.,* nylon), PTFE, etched PTFE, and other thermoplastic elastomers.

The polymer tube 34 can be formed of a single piece of tubing or multiple pieces of tubing. Whether formed of a single piece or multiple pieces, the tube 34 can have a uniform hardness or durometer throughout. Alternatively, different portions of the tube 34 can have different durometers (*e.g.,* the shaft 12 can have a variable durometer from the proximal end 16 to the distal end 18). In an embodiment wherein the tube 34 is formed of multiple pieces, the pieces can be affixed together end to end, or portions of adjacent pieces can overlap each other. These pieces can be coupled or bonded together to form the shaft 12 during a reflow process performed thereon. Additionally, in an exemplary embodiment, one or more portions of the tube 34 disposed at the distal end 18 of the shaft 12, or at any other location on the shaft 12 at or near where an electrode 14 is mounted, are formed so as to be translucent or transparent. The use of transparent or translucent material allows one to locate and access the minor lumen(s) 32 in the outer layer 26 for purposes that will be described in greater detail below.

In an exemplary embodiment, and as illustrated in Figs. 2 and 3, the outer layer 26 further comprises a braided wire assembly 36 disposed adjacent to and between both the inner liner 24 and the polymeric material or tube 34. The arrangement and configuration of the braided wire assembly 36 and the tube 34 is such that the polymeric material of the tube 34 melts and flows into the braid of the braided wire assembly 36 during a reflow process performed on the shaft 12. The braided wire assembly 36, which can extend the entire length of the shaft 12 (*i.e.,* from the proximal end 16 to the distal end 18) or less than the entire length of the shaft 12, maintains the structural integrity of the shaft 12, and also provides an internal member to transfer torque from the proximal end 16 to the distal end 18 of the shaft 12.

In an exemplary embodiment, the braided wire assembly 36 comprises a stainless steel braid wherein each wire of the braid has a rectangular cross-section with the dimensions of 0.002 inches x 0.006 inches (0.051 mm x 0.152 mm). It will be appreciated by those having ordinary skill in the art, however, that the braided wire assembly 36 can be formed of material other than, or in addition to, stainless steel. For example, in another exemplary embodiment, the braided wire assembly 36 comprises a nickel titanium (also known as nitinol) braid. Additionally, the braided wire assembly 36 can have dimensions or wire sizes and cross-sectional shapes other than those specifically provided above, such as, for example, a round or circular cross-sectional shape, and also include varying braid densities throughout. Different braid wire sizes allow different shaft torque and mechanical characteristics. Accordingly, braided wire assemblies comprising materials other than stainless steel, and/or dimensions other than those set forth above, remain within the scope of the present disclosure.

As briefly described above, the outer layer 26 further includes minor lumens 32 disposed therein and coupled thereto. Each minor lumen 32 is adapted to receive and house either an electrical wire(s) associated with an electrode 14, or a deflectable element, such as a pull wire, of the steering mechanism of the sheath 10. The sheath 10 includes extruded tubes 38 (*i.e.,* 38₁-38₈ in Figs. 2 and 3), each one of which defines a corresponding minor lumen 32. The tubes 38, which are also known as spaghetti tubes, can be formed of a number of materials known in the art, such as, for example and without limitation, PTFE. In an exemplary embodiment, the tubes 38 are formed a material having a melting point higher than that of the material in polymer tube 34 so that the tubes 38 will not melt when the shaft 12 is subjected to a reflow process. In the embodiment illustrated in Fig. 2, the tubes 38 are affixed or bonded to the outer surface 30 of the inner layer 24. The tubes 38 can be affixed in a number of ways, such as, for example, using an adhesive. One suitable adhesive is cyanoacrylate. As illustrated in Fig. 3, once the shaft 12 is subjected to a reflow process, the polymeric material of the tube 34 surrounds and encapsulates the tubes 38 resulting in the tubes 38, and therefore the minor lumens 32, being disposed within the outer layer 26.

The minor lumens 32 extend axially relative to the longitudinal axis 22 of the sheath 10. In an exemplary embodiment, some or all of the minor lumens 32 that house electrical wires associated with the electrodes 14 (*i.e.,* lumens 32₂, 32₄, 32₆, 32₈ in Figs. 2 and 3) extend from the proximal end 16 of the shaft 12 to the distal end 18. In another exemplary embodiment, some or all of the minor lumens 32 extend from the proximal end 16 of the shaft 12 to various points or locations on the shaft 12 between the proximal and distal ends 16, 18. For example and with reference to Fig. 1, the minor lumen 32 that houses the electrical wire of the electrode 14₃ can extend from the proximal end 16 of the shaft 12 to the distal end 18. Alternatively, it can extend from the proximal end 16 to the point on the shaft 12 at or near where the electrode 14₃ is mounted. Similarly, minor lumens 32 that house the pull wires of the steering mechanism of the sheath 10 (*i.e.,* the lumens 32₁, 32₃, 32₅, 32₇ in Figs. 2 and 3) can extend from the proximal end 16 of the shaft 12 to the distal end 18. Alternatively, they can extend from the proximal end 16 to a point in the shaft 12 that the pull wire is coupled to another component of the steering mechanism.

In addition to the above, in an exemplary embodiment, the shaft 12 of the sheath 10 can further include a layer 40 of heat shrink material on the outer surface thereof. With continued reference to Figs. 2 and 3, the heat shrink material layer 40 is disposed adjacent to the polymeric material of the outer layer 26 (*e.g.,* the polymer tube 34) such that the outer layer 26 is disposed between the inner liner 24 and the heat shrink material layer 40. The heat shrink material layer 40 can be formed of a number of different types of heat shrink materials. In an exemplary embodiment, the heat shrink material layer 40 comprises a fluoropolymer or polyolefin material, and more particularly, a tube formed of such a material sized to fit over the outer layer 26 of the shaft 16. One example of a suitable material for the heat shrink layer 40 is fluorinated ethylene propylene (FEP).

As will be described in greater detail below, one purpose of the heat shrink material layer 40 relates to the manufacturing process of the sheath 10. More particularly, during manufacture, the shaft 12 is subjected to a heat treating process, such as, for example, a reflow process. During this process, the heat shrink layer 40 is caused to shrink when exposed to a suitable amount of heat. The heat applied to the shaft 12 also causes the polymeric material of the polymer tube 34 to melt, and the shrinking of the heat shrink layer 40 forces the polymeric material to flow into contact with the inner liner 24 and tubes 38 (in an embodiment of the sheath 10 that includes the tubes 38), as well as to flow into the braided wire assembly 36 of the shaft 12 (in an embodiment of the sheath 10 that includes the braided wire assembly 36). In an exemplary embodiment, the heat shrink material layer 40 remains as the outermost layer of the shaft 12. However, in another exemplary embodiment, the heat shrink material layer 40 is removed following the reflow process, and therefore, the polymer tube 34 is the outermost layer of the shaft 12. Accordingly, sheaths 10 that when fully assembled have a heat shrink material layer 40, and sheaths that when fully assembled do not have a heat shrink material layer 40, both remain within the scope of the present disclosure.

In an exemplary embodiment, the shaft 12 can further include a lubricious coating (not shown) that can cover the entire shaft 12 and the electrodes 14 mounted thereon, or just a portion thereof. In an exemplary embodiment, the coating 42 comprises siloxane. However, in other exemplary embodiments, the coating 42 can comprise one of any number of suitable hydrophilic coatings such as, for example, Hydromer® or Hydak® coatings. The purpose of the lubricious coating 42, which can be adjacent to either the polymer tube 34 or the heat shrink layer 40 (if the shaft 12 has a heat shrink layer 40), is to provide the shaft 12 with a smooth and slippery surface that is free of sharp edges, such that the shaft can move with ease when inserted into an anatomical structure.

As briefly described above, and as will be described in greater detail below, the sheath 10 includes one or more electrodes 14 mounted on the shaft 12. As illustrated in Fig. 1, the electrodes 14 can be disposed at or near the distal end 18 of the shaft 14, and can have a number of spacing configurations. In addition, or alternatively, one or more electrodes 14 can be disposed more proximally from the distal end 18. As will be described in greater detail below, in an exemplary embodiment, the shaft 12 is deflectable. In such an embodiment, the electrodes 14 can be mounted on deflectable portions of the shaft 12 and/or non-deflectable portions. In an exemplary embodiment, the electrodes 14 are flush with the outer surface of the shaft 12, and therefore, are recessed into the shaft 12.

The electrodes 14 can comprise any number of types of electrodes and can be used for any number of purposes. For example, the electrodes 14 can comprise one or more of magnetic coil(s), ring electrodes, tip electrodes, or a combination thereof. Further, the electrodes 14 can be used for a number of purposes or to perform one or more functions. For example, the electrodes 14 can be used in the pacing of the heart, monitoring electrocardiograph (ECG) signals, detecting location/position of the electrode 14 and therefore the sheath 10, mapping, visualization of the sheath 10, and the like. Additionally, one or more of the electrodes 14 can be formed of a radiopaque material, such as, for example and without limitation, a metallic material, such as, for example, platinum or another dense material. This permits the visualization of the electrodes 14 by an x-ray based visualization system, such as, for example, a fluoroscopic system. Further, the electrodes 14 can be low impedance electrodes (*e*.*g*., ≤ 600Ω).

According to the present invention, the sheath 10 includes the minor lumens 32 in the outer layer 26 of the shaft 12 and each electrode 14 has one or more elongate electrical conductors or wires 44 associated therewith and electrically coupled thereto. As described above, the sheath 10 includes minor lumens 32 (*i.e*., 32₂, 32₄, 32₆, 328 in Figs. 2 and 3) in the outer layer 26 of the shaft 12 configured to house, for example, the electrical wires 44 associated with the electrodes 14. In an exemplary embodiment, each minor lumen 32 configured to house an electrical wire 44 is configured to house the electrical wire 44 of a single corresponding electrode 14. Accordingly, the electrical wire 44 of a given electrode 14 is electrically connected to the electrode 14, passes through a portion of the outer layer 26 of the shaft 12, and is disposed within the corresponding minor lumen 32. When disposed within the minor lumens 32, the electrical wires 44 are permitted to move within the minor lumen 32 as the shaft 12 is deflected. The minor lumen 32 extends to the proximal end 16 of the shaft 12 such that the electrode wire 44 can be coupled to an interconnect or cable connector (not shown), which allows the electrode 14 to be coupled with other devices, such as a computer, a system for visualization, mapping and/or navigation, and the like. The interconnect is conventional in the art and is disposed at the proximal end 16 of the shaft 12.

Since the sheath 10 comprises minor lumens 32/tubes 38 in the outer layer 26 of the shaft 12 thereof, in an exemplary embodiment, the sheath 10 can be steerable (*i.e.,* the distal end 18 of the shaft 12 can be deflected in one or more directions relative to the longitudinal axis 22 of the sheath 10). In one exemplary embodiment, the movement of the sheath 10 can be controlled and operated manually by a physician. In another exemplary embodiment, however, movement of the sheath 10 can be controlled and operated by an automated guidance system, such as, for example and without limitation, a robotic-based system or a magnetic-based system.

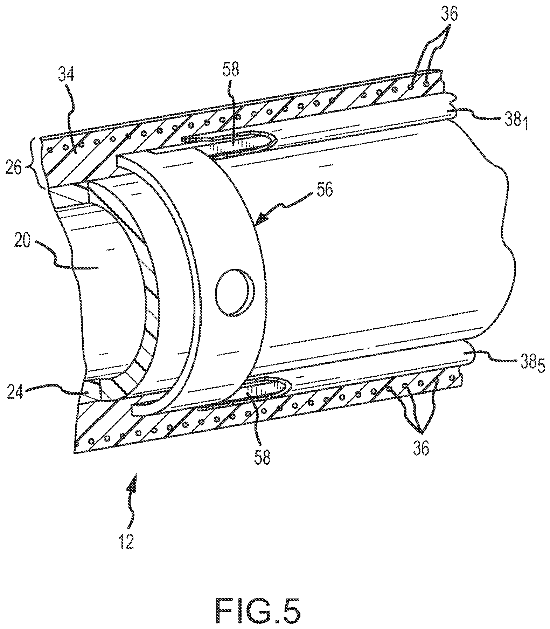

In an exemplary embodiment wherein the sheath 10 is configured for physician control, the sheath 10 includes a steering mechanism 52. A detailed description of an exemplary steering mechanism, such as steering mechanism 52, is set forth in U.S. Patent Publication No. 2007/0299424 entitled "Steerable Catheter Using Flat Pull Wires and Method of Making Same" filed on December 29, 2006. Accordingly, with reference to Figs. 1 and 5, the steering mechanism 52 will be briefly described. In an exemplary embodiment, the steering mechanism 52 comprises a handle 54, a pull ring 56 disposed in the shaft 12 of the sheath 10, and one or deflection elements, such as pull wires 58, coupled with both the handle 54 and the pull ring 56, and disposed within the shaft 12 of the sheath 10.

As illustrated in Fig. 1, the handle 54 is coupled to the shaft 12 at the proximal end 16 thereof. In an exemplary embodiment, the handle 54 provides a location for the physician/clinician to hold the sheath 10 and, in an exemplary embodiment, is operative to, among other things, effect movement (*i.e.,* deflection) of the distal end 18 of the shaft 12 in one or more directions. The handle 54 is conventional in the art and it will be understood that the construction of the handle 54 can vary.

In an exemplary embodiment, the handle 54 includes an actuator 60 disposed thereon or in close proximity thereto, that is coupled to the pull wires 58 of the steering mechanism 52. The actuator 60 is configured to be selectively manipulated to cause the distal end 18 to deflect in one or more directions. More particularly, the manipulation of the actuator 60 causes the pull wires 58 to be pushed or pulled (the length of the pull wires is increased or decreased), thereby effecting movement of the pull ring 56, and thus, the shaft 12. The actuator 60 can take a number of forms known in the art. For example, the actuator 60 can comprise a rotatable actuator, as illustrated in Fig. 1, that causes the sheath 10, and the shaft 12 thereof, in particular, to be deflected in one direction when rotated one way, and to deflect in another direction when rotated in the other way. Additionally, the actuator 60 can control the extent to which the shaft 12 is able to deflect. For instance, the actuator 60 can allow the shaft 12 to deflect to create a soft curve of the shaft. Additionally, or in the alternative, the actuator 60 can allow the shaft 12 to deflect to create a more tight curve (*e.g*., the distal end 18 of the shaft 12 deflects 180 degrees relative to the shaft axis 22. It will be appreciated that while only a rotatable actuator is described in detail here, the actuator 60 can take on any form known the art that effects movement of the distal portion of a sheath or other medical device.

The actuator 60 is coupled to the pull wires 58 of the steering mechanism 52. In an exemplary embodiment, and as with the electrical wires 44 associated with the electrodes 14, the pull wires 58 are located within the outer layer 26 of the shaft 12. More particularly, the pull wires 58 are disposed within minor lumens 32 (*i.e.,* lumens 32₁, 32₃, 32₅, 32₇ in Figs. 2 and 3) in the outer layer 26, and are configured to extend from the handle 54 to the pull ring 56 (best shown in Fig. 5). In an exemplary embodiment, the pull wires 58 have a rectangular cross-section. In other exemplary embodiments, however, the pull wires 58 can have a cross-sectional shape other than rectangular, such as, for example and without limitation, a round or circular cross-sectional shape.

The steering mechanism 52 can comprise a number of different pull wire arrangements. For instance, in the exemplary embodiment illustrated in Figs. 2 and 3, the steering mechanism 52 includes four pull wires 58. In this particular embodiment, the pull wires 58 are disposed 90 degrees apart from each other. In another exemplary embodiment, the steering mechanism comprises two pull wires 58. In such an embodiment, the pull wires 58 are spaced 180 degrees apart from each other.

In either embodiment, the minor lumens 32 within which the electrical wires 44 of the electrodes 14 are housed are located in between the minor lumens 32 for the pull wires 58, and along the neutral axis of the sheath 10. For example, in an exemplary embodiment, there are two pull wires 58, three electrical wires 44, and five minor lumens 32. In such an embodiment, the two minor lumens 32 with the pull wires 58 therein are disposed 180 degrees apart from each other. The remaining three minor lumens 32, each having an electrical wire 44 therein, are placed 90 degrees from each pull wire 58 (e.g., a pair of minor lumens 32 on one side, and one minor lumen 32 on the other). In another exemplary embodiment illustrated, for example, in Figs. 2 and 3, there are four pull wires 58, four electrical wires 44, and eight minor lumens 32. In such an embodiment, the four minor lumens 32 with the pull wires 58 therein (*i.e.,* lumens 32i, 32₃, 32₅, 32₇ in Figs. 2 and 3) are disposed 90 degrees apart from each other. The remaining four minor lumens 32, each having an electrical wire 44 therein (*i.e.,* 32₂, 32₄, 32₆, 32s in Figs. 2 and 3), are placed between each of the four pull wires 58.

The pull wires 58 are coupled at a first end to the actuator 60 and at the second end to the pull ring 56. Fig. 5 is a depiction of a portion of the shaft 12 having the outer layer 26 surrounding the pull ring 56 cut away. As illustrated in Fig. 5, the pull ring 56 is anchored to the shaft 12 at or near the distal end 18 thereof. One exemplary means by which the pull ring 56 is anchored is described in U.S. Patent Publication No. 2007/0199424 entitled "Steerable Catheter Using Flat Pull Wires and Method of Making Same" filed on December 29, 2006. Accordingly, as the pull wires 58 are pulled and/or pushed, the pull wires 58 pull and push the pull ring 56, thereby causing the shaft 12 to move (*e.g*., deflect). Accordingly, the physician manipulates the actuator 60 to cause the distal end 18 of the shaft 12 to move in a certain direction. The actuator 60 pulls and/or pushes the correct pull wires 58, which then causes the pull ring 56, and therefore the shaft 12, to move as directed.

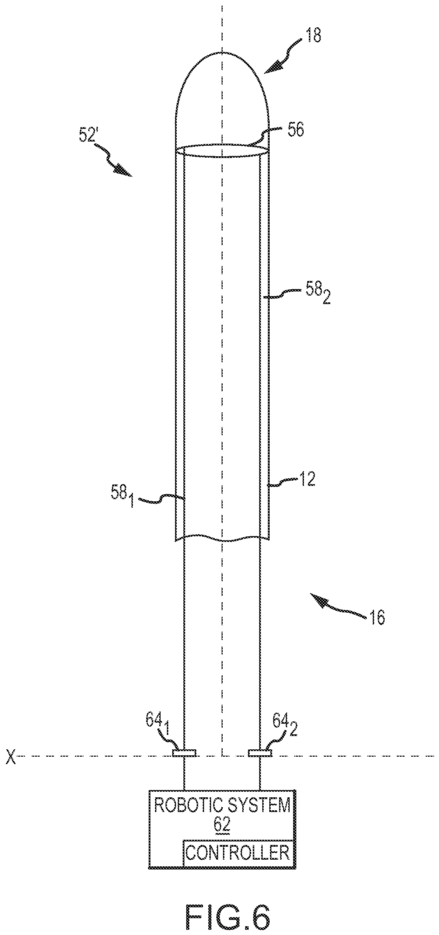

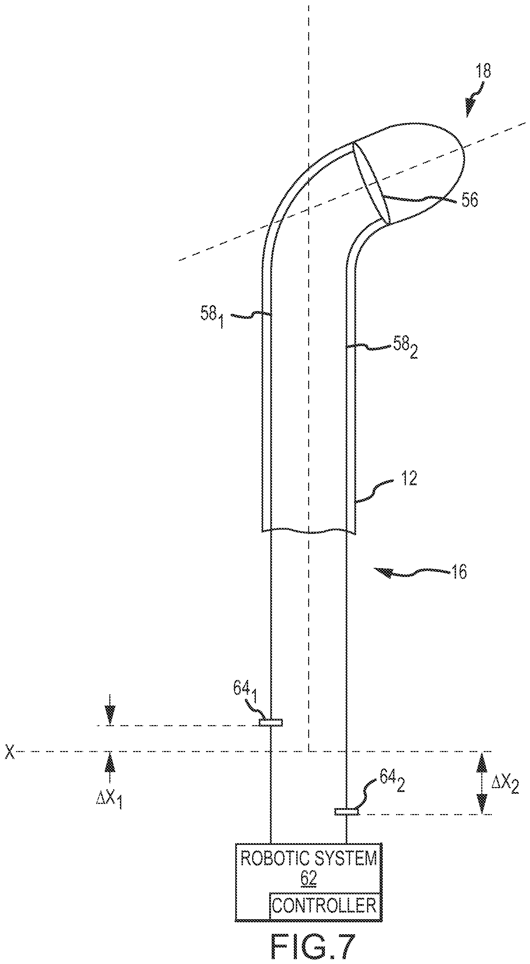

As briefly described above, in another exemplary embodiment, rather than being configured for manual control, the sheath 10 is controlled by an automated guidance system 62. With reference to Figs. 6 and 7, in one exemplary embodiment the automated guidance system 62 is a robotic system (*i.e.,* robotic system 62). In such an embodiment, the sheath 10 includes a steering mechanism 52' that is coupled with the robotic system 62 and acts in concert with, and under the control of, the robotic system 62 to effect movement of the distal end 18 of the shaft 12. Detailed descriptions of exemplary arrangements/configurations by which a robotic system controls the movement of a medical device are set forth in PCT Patent Application Serial No. PCT/2009/038597 entitled "Robotic Catheter System with Dynamic Response" filed on March 27, 2009 (International Publication No. WO/2009/120982), and U.S. Patent Publication No. 2009/0247993 entitled "Robotic Catheter System" filed on December 31, 2008.

To summarize, in an exemplary embodiment, the steering mechanism 52' comprises one or more pull wires 58 (*i.e.,* 58₁ and 58₂ in Figs. 6 and 7) and a pull ring 56. The description above with respect to these components applies here with equal force, and therefore, will not be repeated. However, unlike the embodiment described above, the steering mechanism 52' further comprises one or more control members 64 (*i.e.,* 64₁ and 64₂ in Figs. 6 and 7) equal to the number of pull wires 58, and each control member 64 is affixed or coupled to a respective pull wire 58. The control members 64 are configured to interface or operatively connect control devices, such as, for example, motors or associated linkage or intermediate components thereof, to the pull wires 58. In such an embodiment, the control devices are controlled by a controller, which, in turn, can be fully automated and/or responsive to user inputs relating to the driving or steering of the sheath 10.

In either instance, movement of the control devices (*e.g*., movement of a motor shaft) is translated to cause one or more of the control members 64 to move, thereby resulting in the desired movement of the sheath 10, and the shaft 12 thereof, in particular. For example, Fig. 6 illustrates the shaft 12 in an undeflected state. Thus, both of the control members 64₁, 64₂ are co-located at a position X. However, Fig. 7 illustrated the shaft 12 in a deflected state. In this instance, the control member 64₁ has been pushed toward the distal end 18 of the shaft 12 a distance of ΔX₁, while the control member 64₂ has been pulled away from the distal end 18 of the shaft 12 a distance of ΔX₂. Accordingly, the robotic system 62 is configured to manipulate the positions of the control members 64 of the steering mechanism 52' to effect movement of the shaft 12, and the distal end 18 thereof, in particular.

While the description of an automated sheath control system 62 has been with respect to one particular robotic system, other automated guidance systems and other types of robotic systems can be used. Accordingly, automated guidance systems other than robotic systems, and robotic-based automated guidance systems other than that described with particularity above, remain within the scope of the present disclosure.

It will be appreciated that in addition to the structure of the sheath 10 described above, another aspect of the present disclosure is a method of manufacturing a medical device, such as, for example, the sheath 10. As was noted above, the following description will be limited to an embodiment wherein the medical device is a sheath 10. It will be appreciated, however, that the methodology can be applied to medical devices other than a sheath, and therefore, those medical devices remain within the scope of the present disclosure.

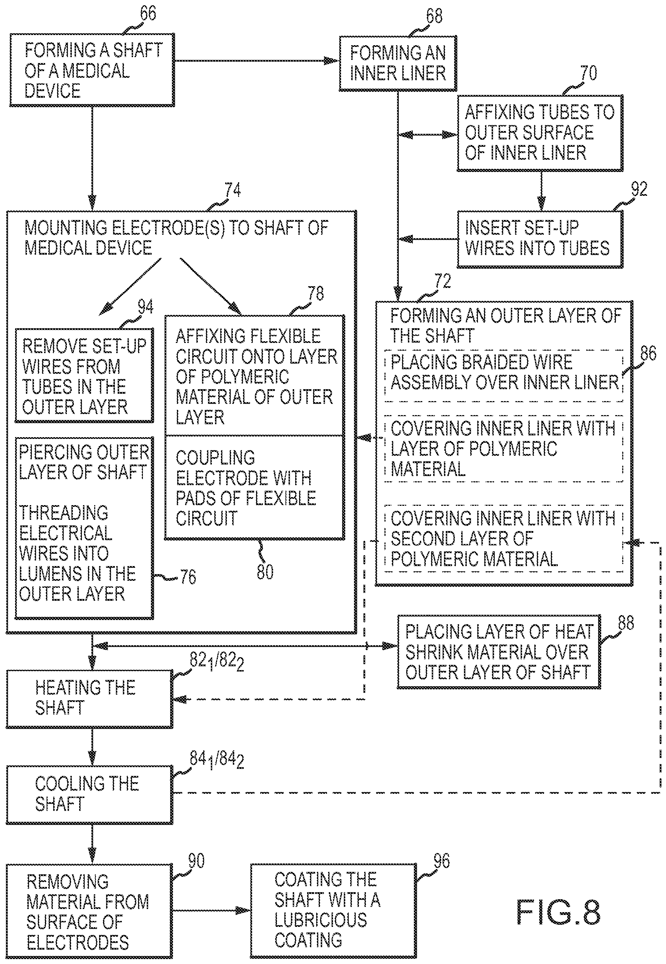

With reference to Fig. 8, in an exemplary example, the method comprises a step 66 of forming a shaft of the sheath 10. The forming a shaft step 66 can comprise a number of substeps. In an exemplary example, a substep 68 comprises forming an inner liner, such as, for example, the inner liner 24 described above. The inner liner 24 has a tubular shape, and has an inner surface 28 and an outer surface 30. In an exemplary example, the inner liner 24 is formed by placing a liner material, such as, for example, etched PTFE, over a mandrel. In this example, the mandrel is removed at or near the end of the manufacturing process, thereby resulting in the creation of the major lumen 20 in the inner liner 24. The inner liner 24 comprises the first layer of the shaft 12 of the sheath 10.

In an exemplary example, the forming step 66 further includes a substep 70 of affixing one or more tubes, such as, for example, the tubes 38 described above, onto the outer surface 30 of the inner liner 24. Each tube 38 defines a minor lumen 32 therein in which, as was described above, a pull wire 58 or an electrical wire 44 is housed. The tubes 38 can be affixed to the outer surface 30 in a number of ways. In an exemplary embodiment, the tubes 38 are affixed using an adhesive, such as, for example, cyanoacrylate.

The forming step 66 still further comprise a substep 72 of forming on outer layer of the shaft 12, such as, for example, the outer layer 26 described above. In an exemplary example, substep 72 comprises covering the inner liner 24 and the tube(s) 38 affixed thereto, if applicable, with one or more layers of polymeric material to form the outer layer 26. For example, in an exemplary example that will be described in greater detail below, the outer layer 26 is formed of two layers of polymeric material. In such an example, the inner liner 24 can be covered with a first layer or tube 34 of polymeric material, and then a second layer or tube 34 of polymeric material. In an example, the second layer of polymeric material is applied after one or more electrodes 14 are mounted onto the shaft 12. The substep 72 can comprise placing one or more tubes formed of a polymeric material, such as the tube 34 described above, over the inner liner 24.

The method yet still further comprises a step 74 of mounting one or more electrodes 14 onto the shaft 12, and onto a layer of polymeric material, in particular. It can be desirable that the sheath 10, and the shaft 12 thereof, in particular, be smooth and free of sharp edges. Accordingly, the mounting step 74 can comprise recessing the electrode(s) into the outer layer 26. In an example, this is done by swaging the outer surface of the electrodes 14 down, thereby forcing the bottom or inner surface of the electrodes 14 down and locking the electrodes 14 into place.

In an example, the electrodes 14 can be mounted to the outer surface of the outer layer 26. However, in as described above, in example, the electrodes 14 are mounted to the shaft 12 after the inner liner 24 is covered with a first layer or tube of polymeric material, and before the inner liner 24 is covered with a second layer or tube of polymeric material. Accordingly, in such an example the electrodes are mounted prior to the completion of the substep 72 of forming the outer layer 26 of the shaft 12.

Since the shaft 12 includes minor lumens 32 therein for housing electrical wires 44 associated with the electrodes 14, the mounting step 74 comprises a substep 76 of threading the electrical wires 44 associated with the electrodes into the corresponding minor lumens 32. Accordingly, the substep 76 is performed for each electrode 14 being mounted to the shaft 12. In an example, the substep 76 comprises piercing or puncturing the outer layer 26 of the shaft 12 at the location at which the electrode 14 is to be mounted to provide access to the distal end of the corresponding minor lumen 32. The electrical wire 44 associated with the electrode 14 is then threaded through the hole in the outer layer 26 and into the minor lumen 32. The electrical wire 44 is then advanced down the minor lumen 32 to the proximal end thereof where the electrical wire 44 can be coupled to an interconnect or connector, such as, for example, the interconnect described above. As the electrical wire 44 is advanced down the minor lumen 32, the electrode 14 is pulled into place on the shaft 12 and covers and seals the access hole through which the electrical wire 44 was inserted. This process is then repeated for each electrode 14 being mounted on the shaft 12. As described above, in an example, once all of the electrodes are mounted to the shaft 12, the shaft 12 and the electrodes 14 are covered with a layer of polymeric material (*i.e.,* a second layer of polymeric material for the outer layer 26), such as, for example, a polymer tube 34, as part of the substep 72 of forming the outer layer 26.

In an example, the method further comprises performing one or more heat treating processes, such as, for example, a reflow process, on at least a portion of the shaft 12, and the outer layer 26 thereof, in particular. Accordingly, in one such example, the method comprises a step 82 of heating the shaft 12 to a temperature at which the polymeric material thereof melts and redistributes around the circumference of the shaft 12. In one example, the temperature applied to the shaft 12 is 400 degrees (F) and the rate of exposure is 1cm/minute. It will be appreciated, however, that temperature and the rate of exposure can vary depending on various factors, such as, for example, the material used. Accordingly, the present disclosure is not meant to be limited to the specific temperature and rate set forth above, and other temperatures and rates remain within the scope of the present disclosure.

In an example, multiple heating steps are performed on the shaft 12 at multiple points in the manufacturing process. For example, in the examples described above wherein the outer layer 26 comprises two layers of polymeric material, two heating processes are performed. More particularly, after the inner liner 24 is covered with the first layer or tube 34, a first heating step 82₁ is performed. After the application of a second layer or tube 34 over said inner liner 24, a second heating step 82₂ is performed.

Once the heating step 82 is complete, a step 84 of cooling the shaft 12, and therefore, the polymeric material, is performed. In an exemplary example, the cooling step 84 comprises letting the shaft 12 air-cool. However, in another example, a cooling process can be performed on the shaft 12.

As with the heating step described above, in an example, multiple cooling steps are performed on the shaft 12 at multiple points in the manufacturing process. For instance, in an embodiment wherein the outer layer 26 comprises two layers of polymeric material or tubes 34, a first cooling step 84₁ is performed after the first layer or tube 34 is heated. After the second layer or tube 34 is heated, a second cooling step 84₂ is performed.

In an example, prior to covering the inner liner 24 with polymeric material, the forming an outer layer of the shaft substep 72 further comprises a substep 86 of placing a braided wire assembly, such as the braided wire assembly 36 described above, over the inner liner 24 and the tubes 38, if applicable. In such an example, once the substep 86 is complete, the substep(s) of covering the inner liner 24 with a polymeric material is performed. Therefore, the combination of the braided wire assembly 36 and the polymeric material comprises the outer layer 26.

In an example, and prior to performing the heating step 82, the method further comprises a step 88 of placing a layer of heat shrink material, such as, for example, the heat shrink material layer 40 described above, over the outer layer 26 of the shaft 12. The heat shrink material layer 40 is formed of a material that has a higher melt temperature than that of the polymeric material of the outer layer 26 such that when the heating step 82 is performed, the heat shrink material layer 40 retains it tubular shape and forces the polymeric material into the braided wire assembly 36 (if the shaft 12 comprises a braided wire assembly 36), and into contact with the inner liner 24, tubes 38, and/or flexible circuit 46 (depending on the construction and composition of the shaft 12), but does not itself melt. In an exemplary embodiment, following the heating step 82 and either during or following the cooling step 84, the heat shrink material layer 40 is removed. Alternatively, the heat shrink material layer 40 is not removed, but rather remains as part of the shaft 12.

In certain example, the electrodes 14 can be covered with one or more layers of material, such as, for example, polymeric material or heat shrink material. This can be because the electrodes 14 were covered with a layer of polymeric material during the formation of the outer layer 26, or because polymeric material migrated onto the surface of the electrodes 14 during a heating process performed on the shaft 12. In either instance, the method further comprises a step 90 of removing the material from the outer surface of the electrodes 14. Step 90 can be performed in a number of ways, such as, for exemplary purposes only, laser ablating the material away from the surface of the electrodes 14. It will be appreciated by those having ordinary skill in the art, however, that other known processes or techniques can be used to remove the material, and those processes or techniques remain within the scope of the present disclosure.

In addition to the description above, since the shaft 12 includes the minor lumens 32 therein, the method can further comprise a step 92 of inserting set-up wires into one or more of the minor lumens 32 defined by the tubes 38. The purpose of inserting set-up wires in the minor lumens 32 is to prevent the tubes 38 from collapsing during the subsequent steps of the manufacturing process. Accordingly, either prior to tubes 38 being affixed to the outer surface 30 of the inner liner 24 or after the tubes 38 are affixed, set-up wires are inserted into the minor lumens 32. Following the performance of one or more heat treating processes on the shaft 12, in a step 94, the set-up wires are removed from the minor lumens 32 and replaced with the electrical wires 44.

In an example, following the cooling step 84 and/or the removal step 90, the method further comprises a step 96 of coating the outer surface of the shaft 12, and in an example the outer surface of the electrodes 14 as well, with a lubricious coating, such as, for example, the lubricious coating described above.

In accordance with another aspect of the disclosure, the sheath 10 is part of a system 98 for performing one or more diagnostic or therapeutic medical procedures, such as, for example and without limitation, drug delivery, the pacing of the heart, pacer lead placement, tissue ablation, monitoring, recording, and/or mapping of electrocardiograph (ECG) signals and other electrophysiological data, and the like. In addition to the sheath 10, the system 98 comprises, at least in part, a system 100 for visualization, mapping, and/or navigation of internal body structures and medical devices. In an exemplary embodiment, the system 100 includes an electronic control unit (ECU) 102 and a display device 104. In another exemplary embodiment, the display device 104 is separate and distinct from the system 100, but electrically connected to and configured for communication with the ECU 102.

As will be described in greater detail below, one purpose of the system 100 is to accurately determine the position and orientation of the sheath 10, and in certain embodiments, to accurately display the position and orientation of the sheath 10 for the user to see. Knowing the position and orientation of the sheath 10 is beneficial regardless of whether the sheath is manually controlled (*i.e.,* by a physician or clinician) or controlled by an automated guidance system, such as, for example, a robotic-based or magnetic-based system. For example, in a robotic-based system, it is important to know the accurate position and orientation of the sheath 10 to minimize error and provide patient safety by preventing perforations to the cardiac tissue. In a magnetic-based systems, it is important for the physician/clinician operating the system to know the accurate location and orientation of, for example, the fulcrum of a catheter used with the sheath 10. This information allows the physician/clinician to direct the orientation of the sheath 10 to optimize the ability to locate the catheter precisely and take full advantage of the magnetic manipulation capability offered by magnetic-based systems.

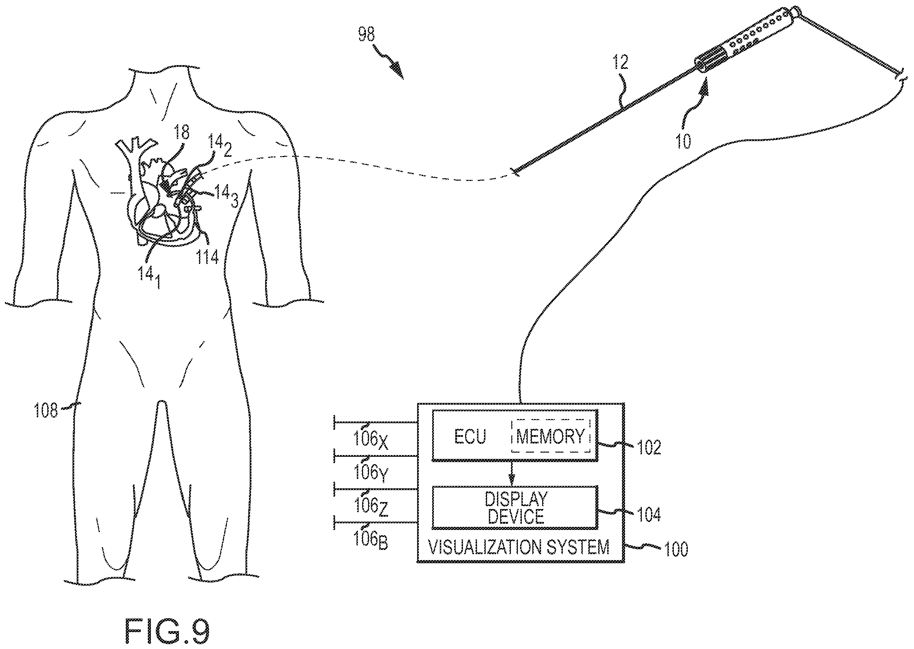

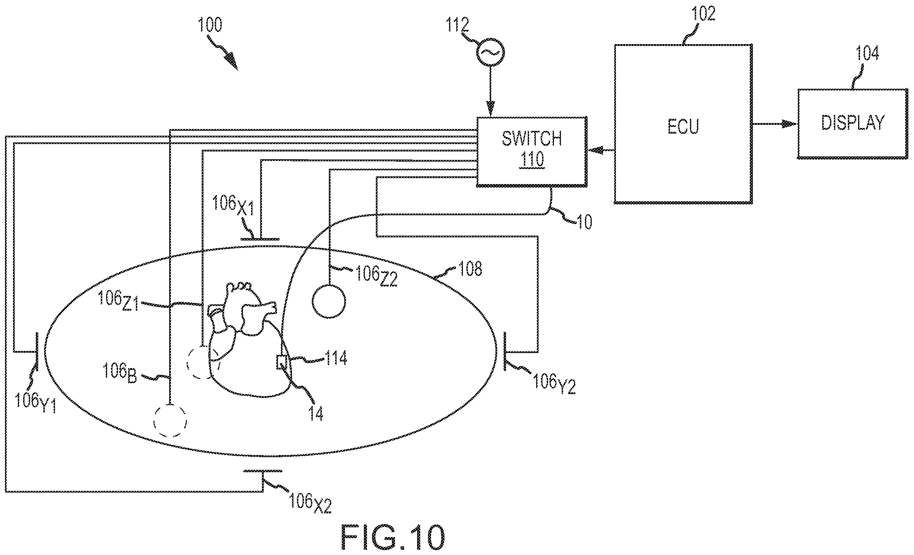

With reference to Figs. 9 and 10, the visualization, navigation, and/or mapping system 100 will be described. The system 100 can comprise an electric field-based system, such as, for example, the EnSite NavX™ system commercially available from St. Jude Medical, Inc., and as generally shown with reference to U.S. Patent No. 7,263,397 entitled "Method and Apparatus for Catheter Navigation and Location and Mapping in the Heart,". In other exemplary embodiments, however, the system 100 can comprise systems other than electric field-based systems. For example, the system 100 can comprise a magnetic field-based system such as the Carto™ system commercially available from Biosense Webster, and as generally shown with reference to one or more of U.S. Patent Nos.: 6,498,944 entitled "Intrabody Measurement;" 6,788,967 entitled "Medical Diagnosis, Treatment and Imaging Systems;" and 6,690,963 entitled "System and Method for Determining the Location and Orientation of an Invasive Medical Instrument,". In another exemplary embodiment, the system 100 comprises a magnetic field-based system such as the gMPS system commercially available from MediGuide Ltd., and as generally shown with reference to one or more of U.S. Patent Nos.: 6,233,476 entitled "Medical Positioning System;" 7,197,354 entitled "System for Determining the Position and Orientation of a Catheter;" and 7,386,339 entitled "Medical Imaging and Navigation System,". In yet another embodiment, the system 100 can comprise a combination electric field-based and magnetic field-based system, such as, for example and without limitation, the Carto 3™ system also commercially available from Biosense Webster, and as generally shown with reference to U.S. Patent No. 7,536,218 entitled "Hybrid Magnetic-Based and Impedance Based Position Sensing,". In yet still other exemplary embodiments, the system 100 can comprise or be used in conjunction with other commonly available systems, such as, for example and without limitation, fluoroscopic, computed tomography (CT), and magnetic resonance imaging (MRI)-based systems. For purposes of clarity and illustration only, the system 100 will be described hereinafter as comprising an electric field-based system.

As illustrated in Figs. 9 and 10, in addition to the ECU 102 and the display 104, in an exemplary embodiment the system 100 further comprises a plurality of patch electrodes 106. With the exception of the patch electrode 106_{B} called a "belly patch," the patch electrodes 106 are provided to generate electrical signals used, for example, in determining the position and orientation of the sheath 10, and potentially in the guidance thereof. In one embodiment, the patch electrodes 106 are placed orthogonally on the surface of a patient's body 108 and used to create axes-specific electric fields within the body 108. For instance, in one exemplary embodiment, the patch electrodes 106ₓ₁, 106ₓ₂ can be placed along a first (x) axis. The patch electrodes 106_{y1}, 106_{y2} can be placed along a second (y) axis. Finally, the patch electrodes 106_{z1}, 106_{z2} can be placed along a third (z) axis. Each of the patch electrodes 106 can be coupled to a multiplex switch 110. In an exemplary embodiment, the ECU 102 is configured through appropriate software to provide control signals to switch 110 to thereby sequentially couple pairs of electrodes 106 to a signal generator 112. Excitation of each pair of electrodes 106 generates an electric field within the body 108 and within an area of interest such as, for example, heart tissue 114. Voltage levels at non-excited electrodes 106, which are referenced to the belly patch 106_{B}, are filtered and converted, and provided to the ECU 102 for use as reference values.

As described above, the sheath 10 includes one or more electrodes 14 mounted thereon. In an exemplary embodiment, one of the electrodes 14 is a positioning electrode (however, in another exemplary embodiment, a plurality of the electrodes 14 are positioning electrodes). The positioning electrode 14 can comprise, for example and without limitation, a ring electrode or a magnetic coil sensor. The positioning electrode 14 is placed within electric fields created in the body 108 (*e.g.*, within the heart) by exciting patch electrodes 106. The positioning electrode 14 experiences voltages that are dependent on the location between the patch electrodes 106 and the position of the positioning electrode 14 relative to the heart tissue 114. Voltage measurement comparisons made between the electrode 14 and the patch electrodes 106 can be used to determine the position of the positioning electrode 14 relative to the heart tissue 114. Movement of the positioning electrode 14 proximate the heart tissue 114 (*e.g*., within a heart chamber, for example) produces information regarding the geometry of the tissue 114. This information can be used, for example and without limitation, to generate models and maps of tissue or anatomical structures. Information received from the positioning electrode 14 (or if multiple positioning electrodes, the positioning electrodes 14) can be used to display on a display device, such as display device 104, the location and orientation of the positioning electrode 14 and/or the distal end of the sheath 10, and the shaft 12 thereof, in particular, relative to the tissue 114. Accordingly, among other things, the ECU 102 of the system 100 provides a means for generating display signals used to control the display device 104 and the creation of a graphical user interface (GUI) on the display device 104.

Accordingly, the ECU 102 can provide a means for determining the geometry of the tissue 114, EP characteristics of the tissue 114, and the position and orientation of the sheath 10. The ECU 102 can further provide a means for controlling various components of the system 100, including, without limitation, the switch 110. It should be noted that while in an exemplary embodiment the ECU 102 is configured to perform some or all of the functionality described above and below, in another exemplary embodiment, the ECU 102 can be a separate and distinct component from the system 100, and the system 100 can have another processor configured to perform some or all of the functionality (*e.g*., acquiring the position/location of the positioning electrode/sheath, for example). In such an embodiment, the processor of the system 100 would be electrically coupled to, and configured for communication with, the ECU 102. For purposes of clarity only, the description below will be limited to an embodiment wherein the ECU 102 is part of the system 100 and configured to perform all of the functionality described herein.

The ECU 102 can comprise a programmable microprocessor or microcontroller, or can comprise an application specific integrated circuit (ASIC). The ECU 102 can include a central processing unit (CPU) and an input/output (I/O) interface through which the ECU 102 can receive a plurality of input signals including, for example, signals generated by patch electrodes 106 and the positioning electrode 14, and generate a plurality of output signals including, for example, those used to control and/or provide data to the display device 104 and the switch 110. The ECU 102 can be configured to perform various functions, such as those described in greater detail below, with appropriate programming instructions or code (*i.e.,* software). Accordingly, the ECU 102 is programmed with one or more computer programs encoded on a computer storage medium for performing the functionality described herein.

In operation, the ECU 102 generates signals to control the switch 110 to thereby selectively energize the patch electrodes 106. The ECU 102 receives position signals (location information) from the sheath 10 (and particularly the positioning electrode 14) reflecting changes in voltage levels on the positioning electrode 14 and from the non-energized patch electrodes 106. The ECU 102 uses the raw location data produced by the patch electrodes 106 and positioning electrode 14 and corrects the data to account for respiration, cardiac activity, and other artifacts using known or hereinafter developed techniques. The ECU 102 can then generate display signals to create an image or representation of the sheath 10 that can be superimposed on an EP map of the tissue 114 generated or acquired by the ECU 102, or another image or model of the tissue 114 generated or acquired by the ECU 102.

In an embodiment wherein there are multiple positioning electrodes 14, the ECU 102 can be configured to receive positioning signals from two or more of the positioning electrodes 14, and to then create a representation of the profile of the distal portion of the sheath 10, for example, that can be superimposed onto an EP map of the tissue 114 generated or acquired by the ECU 102, or another image or model of the tissue 114 generated or acquired by the ECU 102.

One example where this functionality is valuable relates to the treatment of atrial fibrillation. In atrial fibrillation, often the left side of the heart has to be accessed. Using a technique called transseptal access, the physician uses a long, small diameter needle to pierce or puncture the heart's septal wall in an area known as the fossa ovalis to provide a means of access from the right atrium to the left atrium. Once transseptal access is obtained, physicians prefer not to lose it. However, for a variety of reasons, there are times when the access to the left side through the fossa ovalis is lost. As a result, the procedure time is increased and additional piercing or puncturing of the septal wall can be required.

If multiple positioning electrodes are mounted on the sheath, however, using the system 102 the location of the positioning electrodes 14, and therefore, the sheath 10 can be determined, and a shadow representation of the sheath 10 can be superimposed onto an image or model of the tissue 114 showing its position across the fossa ovalis. This gives the physician a reference to use as guidance, and more particularly, permits the physician to reposition the sheath 10 in the same location as the shadow representation, should access to the left side be lost during the procedure. Thus, additional piercing or puncturing of the septal wall can be avoided, the speed of the procedure will be reduced, and fluoroscopy time can also be reduced. Further, the positioning electrodes 14 can be used in real time to "straddle" the fossa ovalis so as to allow the physician to try to prevent the sheath 10 from coming out of the fossa ovalis in the first place.

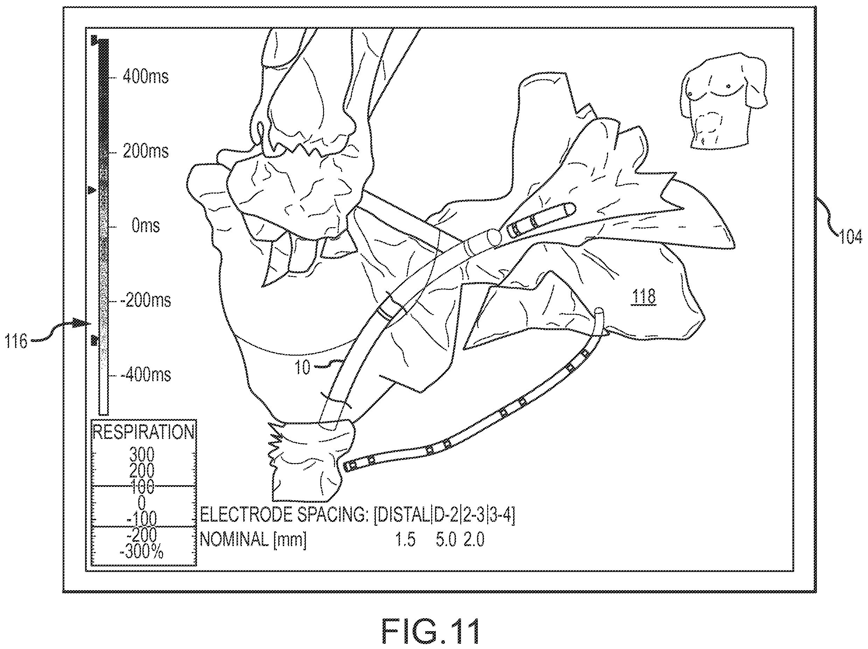

With reference to Figs. 9 and 11, the display device 104, which, as described above, can be part of the system 100 or a separate and distinct component, is provided to convey information to a clinician to assist in, for example, the performance of therapeutic or diagnostic procedures on the tissue 114. The display device 104 can comprise a conventional computer monitor or other display device known in the art. With particular reference to Fig. 11, the display device 104 presents a graphical user interface (GUI) 116 to the clinician. The GUI 116 can include a variety of information including, for example and without limitation, an image or model of the geometry of the tissue 114, EP data associated with the tissue 114, electrocardiograms, electrocardiographic maps, and images or representations of the sheath 10 and/or positioning electrode 14. Some or all of this information can be displayed separately (*i.e.,* on separate screens), or simultaneously on the same screen. The GUI 116 can further provide a means by which a clinician can input information or selections relating to various features of the system 100 into the ECU 102.

The image or model of the geometry of the tissue 114 (image/model 118 shown in Fig. 11) can comprise a two-dimensional image of the tissue 108 (*e.g.,* a cross-section of the heart) or a three-dimensional image of the tissue 114. The image or model 118 can be generated by the ECU 102 of the system 100, or alternatively, can be generated by another imaging, modeling, or visualization system (*e.g.*, fluoroscopic, computed tomography (CT), magnetic resonance imaging (MRI), etc. based systems) that are communicated to, and therefore, acquired by, the ECU 102. As briefly mentioned above, the display device 104 can also include an image or representation of the sheath 10 and/or the positioning electrode 14 illustrating their position and orientation relative to the tissue 114. The image or representation of the sheath 10 can be part of the image 118 itself (as is the case when, for example, a fluoroscopic system is used) or can be superimposed onto the image/model 118.

It will be appreciated that as briefly described above, in an exemplary embodiment, one or more of the electrodes 14 mounted on the shaft 12 can be used for purposes other than for determining positioning information. For example, one or more electrodes can be used for pacing in the atrium of the heart to, for example, determine bi-directional block on the septal wall.

In addition, or alternatively, one or more of the electrodes 14 can be used for monitoring electrocardiographs or to collect EP data in one or more areas in the heart. The information or data represented by the signals acquired by these electrodes 14 can be stored by the ECU 102 (*e.g.,* in a memory of the device, for example), and/or the ECU 102 can display the data on an EP map or another image/model generated or acquired by the ECU 102, or otherwise display the data represented by the signals acquired by the electrodes 14 on a display device such as, for example, the display device 104. For example, in an exemplary embodiment, one or more electrodes 14 can be positioned such that as a therapeutic procedure is being performed on the left side of the fossa ovalis, ECGs or other EP data can be monitored on both the left and right sides of the fossa ovalis using the electrodes 14. One benefit of such an arrangement is that fewer medical devices need to be used during a procedure.

Accordingly, the system 98, and the visualization, navigation, and/or mapping system 100 thereof, in particular, is configured to carry out and perform any number of different functions, all of which remain within the scope of the present disclosure.

It should be understood that the system 100, and particularly the ECU 102 as described above, can include conventional processing apparatus known in the art, capable of executing pre-programmed instructions stored in an associated memory, all performing in accordance with the functionality described herein. It is contemplated that the methods described herein, including without limitation the method steps of embodiments of the disclosure, will be programmed in a preferred embodiment, with the resulting software being stored in an associated memory and where so described, can also constitute the means for performing such methods. Implementation of the disclosure, in software, in view of the foregoing enabling description, would require no more than routine application of programming skills by one of ordinary skill in the art. Such a system can further be of the type having both ROM, RAM, a combination of non-volatile and volatile (modifiable) memory so that the software can be stored and yet allow storage and processing of dynamically produced data and/or signals.

Although only certain embodiments have been described above with a certain degree of particularity, those skilled in the art could make numerous alterations to the disclosed embodiments without departing from the scope of this disclosure. Joinder references (*e.g*., attached, coupled, connected, and the like) are to be construed broadly and can include intermediate members between a connection of elements and relative movement between elements. As such, joinder references do not necessarily infer that two elements are directly connected/coupled and in fixed relation to each other. Additionally, the terms electrically connected and in communication are meant to be construed broadly to encompass both wired and wireless connections and communications. It is intended that all matter contained in the above description or shown in the accompanying drawings shall be interpreted as illustrative only and not limiting. Changes in detail or structure can be made without departing from the disclosure as defined in the appended claims.

## Claims

1. A medical device (10) comprising:
a catheter-introducer (12) having a proximal end (16), a distal end (18) and a central major lumen (20) extending through said catheter-introducer (12) between the proximal end (16) and the distal end (18) along a longitudinal axis (22);
said catheter-introducer (12) comprising an inner liner (24) and an outer layer (26) adjacent said inner liner (24),
wherein said inner liner (24) comprises an inner surface (28) and an outer surface (30), said inner surface (28) surrounding the central lumen (20),
wherein said central lumen (20) is adapted to receive a second medical device therethrough;
a plurality of extruded tubes (38, 38₁-38₈), each one of which defining a corresponding minor lumen (32, 32₁-32₈) extending through said catheter-introducer (12) intermediate the inner liner (24) and the outer layer (26);
an electroanatomical system imaging element (14) coupled to a distal portion of said catheter-introducer (12);
a plurality of electrical wires (44) coupled to said electroanatomical system imaging element (14), wherein each of said plurality of electrical wires (44) extends respectively through one of said minor lumens (32, 32₁-32₈);
at least one deflection element (58), wherein each of said at least one deflection element (58) extends respectively through another one of said minor lumens (32, 32₁-32₈); and
an actuator (60) coupled to the at least one deflection member (58) and capable of deflecting the distal end (18) of the catheter-introducer (12).

2. The medical device (10) according to claim 1, wherein each of said plurality of extruded tubes (38, 38₁-38₈) is coupled to said outer surface (30), preferably wherein an extruded tube (38, 38₁-38₈) inner diameter is less than an inner liner (24) inner diameter and an extruded tube (38, 38₁-38₈) outer diameter is less than an inner liner (24) outer diameter.

3. The medical device (10) according to claim 1, wherein said electroanatomical system imaging element (14) is a first electroanatomical system imaging element further comprising:
a second electroanatomical system imaging element coupled to said catheter-introducer (12); and
a third electroanatomical system imaging element coupled to said catheter-introducer (12), preferably wherein said first electroanatomical system imaging element, said second electroanatomical system imaging element, and said third electroanatomical system imaging element are operably connected to an electroanatomical navigation system (100).

4. The medical device (10) according to claim 1, further comprising a layer (40) of heat shrink material adjacent said outer layer (26) such that said outer layer (26) couples to the inner liner (24) and the layer (40) of heat shrink material.

5. The medical device (10) according to claim 1, wherein at least a portion of a distal end of said outer layer (26) comprises at least one of a transparent polymeric material and a translucent polymeric material, preferably wherein a distal end of said plurality of extruded tubes (38, 38₁-38₈) is adjacent said portion of said outer layer (26) comprising at least one of a transparent polymeric material and a translucent polymeric material.

6. The medical device (10) according to claim 1, wherein said electroanatomical system imaging element (14) comprises at least one of: an impedance-measuring electrode element, a magnetic field sensor element, an acoustic-ranging system element, a conductive coil element, a computed tomography imaging element, and a magnetic resonance imaging element.

7. The medical device (10) according to claim 1, wherein said electroanatomical system imaging element (14) comprises one of: a pacing electrode, an electrophysiological electrode, a positioning electrode, a mapping electrode, a ring electrode, a solid electrode, and a tip electrode.

8. A system (98) comprising:
a catheter-introducer (12) as defined in claim 1, and
an electroanatomical navigation system (100) adapted to receive signals from said electroanatomical system imaging element (14).

9. The system (98) according to claim 8, wherein said electroanatomical navigation system (100) comprises means for performing at least one of determining a position of said electroanatomical system imaging element (14) and monitoring an electrophysiological signal.

10. The system (98) according to claim 9, wherein said means for determining a position comprises at least one of: an impedance-measuring electrode, a magnetic field sensor element, an acoustic-ranging system element, a conductive coil element, a computed tomography imaging element, and a magnetic resonance imaging element.

11. The system (98) according to claim 8, wherein said electroanatomical navigation system (100) comprises a display screen (104), wherein said display screen (104) is adapted for displaying a visual representation of a three-dimensional location of one or more catheters.

12. The system (98) according to claim 9, further comprising a second medical device capable of delivering or receiving energy.

## Patentansprüche

1. Medizinische Einrichtung (10), enthaltend:
einen Katheter-Einführer (12) mit einem nahen Ende (16), einem entfernten Ende (18) und einem mittigen Hauptlumen (20), das sich durch den Katheder-Einführer (12) zwischen dem nahen Ende (16) und dem entfernten Ende (18) entlang einer Längsachse (22) erstreckt;
wobei der Katheter-Einführer (12) eine Innenauskleidung (24) und eine Außenschicht (26) an die Innenauskleidung (24) angrenzend aufweist,
wobei die Innenauskleidung (24) eine Innenfläche (28) und eine Außenfläche (30) aufweist, wobei die Innenfläche (28) das mittige Lumen (20) umgibt,
wobei das mittige Lumen (20) derart angepasst ist, dass es eine zweite medizinische Einrichtung dort hindurch aufnimmt;
eine Vielzahl von extrudierten Rohren (38, 38₁-38₈), von denen jedes ein entsprechendes geringes Lumen (32, 32₁-32₈) definiert, das sich durch den Katheter-Einführer (12) zwischen der Innenauskleidung (24) und der Außenschicht (26) liegend erstreckt;
ein Elektroanatomisches-System-Abbildungselement (14), das mit einem entfernten Bereich des Katheter-Einführers (12) gekoppelt ist;
eine Vielzahl von elektrischen Leitungen (44), die mit dem Elektroanatomisches-System-Abbildungselement (14) gekoppelt sind, wobei jede der Vielzahl von elektrischen Leitungen (44) sich entsprechend durch eines der geringen Lumen (32, 32₁-32₈) erstreckt;
wenigstens ein Auslenkungselement (58), wobei jedes aus dem wenigstens einen Auslenkungselement (58) sich entsprechend durch ein anderes aus den geringen Lumen (32, 32₁-32₈) erstreckt, und
einen Aktuator (6), der mit dem wenigstens einen Auslenkungselement (58) gekoppelt ist und geeignet ist zum Auslenken des entfernten Endes (18) des Katheter-Einführers (12).

2. Medizinische Einrichtung (10) nach Anspruch 1, wobei jedes aus der Vielzahl von extrudierten Rohren (38, 38₁-38₈) mit der Außenfläche (30) gekoppelt ist, wobei bevorzugt ein Innendurchmesser eines extrudierten Rohres (38, 38₁-38₈) geringer ist als ein Innendurchmesser der Innenauskleidung (24) und ein Außendurchmesser eines extrudierten Rohres (38, 38₁-38₈) geringer ist als ein Außendurchmesser der Innenauskleidung (24).

3. Medizinische Einrichtung (10) nach Anspruch 1, wobei das Elektroanatomisches-System-Abbildungselement (14) ein erstes Elektroanatomisches-System-Abbildungselement ist, das weiter aufweist:
ein zweites Elektroanatomisches-System-Abbildungselement, das mit dem Katheter-Einführer (12) gekoppelt ist; und
ein drittes Elektroanatomisches-System-Abbildungselement, das mit dem Katheter-Einführer (12) gekoppelt ist, wobei das erste Elektroanatomisches-System-Abbildungselement das zweite Elektroanatomisches-System-Abbildungselement und das dritte Elektroanatomisches-System-Abbildungselement bevorzugt betriebsbereit mit einem elektroanatomischen Navigationssystem (100) verbunden sind.

4. Medizinische Einrichtung (10) nach Anspruch 1, ferner enthaltend eine Schicht (40) aus einem Wärmeschrumpfmaterial, das an die Außenschicht (26) derart angrenzt, dass die Außenschicht (26) mit der Innenauskleidung (24) und der Schicht (40) aus einem Wärmeschrumpfmaterial gekoppelt ist.

5. Medizinische Einrichtung (10) nach Anspruch 1, wobei wenigstens ein Bereich eines entfernten Endes der Außenschicht (26) wenigstens eines aus einem transparenten polymerischen Material und einem durchscheinenden polymerischen Material aufweist, wobei ein entferntes Ende der Vielzahl von extrudierten Rohren (38, 381-388) bevorzugt an den Bereich der Außenschicht (26) angrenzt, der wenigstens eines aus einem transparenten polymerischen Material und einem durchscheinenden polymerischen Material aufweist.

6. Medizinische Einrichtung (10) nach Anspruch 1, wobei das Elektroanatomisches-System-Abbildungselement (14) wenigstens eines aufweist aus: ein einen Widerstand messendes Elektrodenelement, ein Magnetfeldsensorelement, ein einen Schall messendes Systemelement, ein konduktives Spulenelement, ein Computertomografie-Abbildungselement und ein Magnetwiderstand-Abbildungselement.

7. Medizinische Einrichtung (10) nach Anspruch 1, wobei das Elektroanatomisches-System-Abbildungselement (14) eines aufweist aus: eine Stimulationselektrode, eine elektrophysiologische Elektrode, eine Positionselektrode, eine Kartografieelektrode, eine Ringelektrode, eine Festkörperelektrode und eine Spitzenelektrode.

8. System (98), enthaltend:
einen Katheter-Einführer (12) nach Anspruch 1, und
ein elektroanatomisches Navigationssystem (100), das zum Empfangen von Signalen von dem Elektroanatomisches-System-Abbildungselement (14) angepasst ist.

9. System (98) nach Anspruch 8, wobei das elektroanatomische Navigationssystem (100) Mittel zum Durchführen von wenigstens einem aus Bestimmen einer Position des Elektroanatomisches-System-Abbildungselements (14) und Beobachten eines elektrophysiologischen Signals aufweist.

10. System (98) nach Anspruch 9, wobei das Mittel zum Bestimmen einer Position wenigstens eines aufweist aus: ein einen Widerstand messendes Elektrodenelement, ein Magnetfeldsensorelement, ein einen Schall messendes Systemelement, ein konduktives Spulenelement, ein Computertomografie-Abbildungselement und ein Magnetwiderstand-Abbildungselement.

11. System (98) nach Anspruch 8, wobei das elektroanatomische Navigationssystem (100) einen Bildschirm (104) aufweist, wobei der Bildschirm (104) angepasst ist zum Anzeigen einer bildlichen Darstellung eines dreidimensionalen Orts eines oder mehrerer Katheter.

12. System (98) nach Anspruch 9, ferner enthaltend eine zweite medizinische Einrichtung, die geeignet ist zum Abgeben oder Empfangen von Energie.

## Revendications

1. Dispositif médical (10) comprenant :
un introducteur de cathéter (12) ayant une extrémité proximale (16), une extrémité distale (18) et une lumière principale centrale (20) s'étendant à travers ledit introducteur de cathéter (12) entre l'extrémité proximale (16) et l'extrémité distale (18) le long d'un axe longitudinal (22) ;
ledit introducteur de cathéter (12) comprenant un revêtement interne (24) et une couche externe (26) adjacente audit revêtement interne (24) ;
dans lequel ledit revêtement interne (24) comprend une surface interne (28) et une surface externe (30), ladite surface interne (28) entourant la lumière centrale (20),
dans lequel ladite lumière centrale (20) est adaptée pour recevoir un second dispositif médical à travers elle ;
une pluralité de tubes extrudés (38, 38₁-38₈), chacun définissant une lumière secondaire correspondante (32, 32₁-32₈) s'étendant à travers ledit introducteur de cathéter (12) entre le revêtement interne (24) et la couche externe (26) ;
un élément d'imagerie de système électroanatomique (14) couplé à une partie distale dudit introducteur de cathéter (12) ;
une pluralité de fils électriques (44) couplés audit élément d'imagerie de système électroanatomique (14), dans lequel chacun de ladite pluralité de fils électriques (44) s'étend respectivement à travers l'une desdites lumières secondaires (32, 32₁-32₈) ;
au moins un élément de déviation (58), dans lequel chacun desdits au moins un élément de déviation (58) s'étend respectivement à travers une autre desdites lumières secondaires (32, 32₁-32₈) ; et
un actionneur (60) couplé à l'au moins un élément de déviation (58) et capable de dévier l'extrémité distale (18) de l'introducteur de cathéter (12).

2. Dispositif médical (10) selon la revendication 1, dans lequel chacun de ladite pluralité de tubes extrudés (38, 38₁-38₈) est couplé à ladite surface externe (30), de préférence dans lequel un diamètre interne du tube extrudé (38, 38₁-38₈) est inférieur à un diamètre interne du revêtement interne (24) et un diamètre externe du tube extrudé (38, 38₁-38₈) est inférieur à un diamètre externe du revêtement interne (24).

3. Dispositif médical (10) selon la revendication 1, dans lequel ledit élément d'imagerie de système électroanatomique (14) est un premier élément d'imagerie de système électroanatomique comprenant en outre :
un second élément d'imagerie de système électroanatomique couplé audit introducteur de cathéter (12) ; et
un troisième élément d'imagerie de système électroanatomique couplé audit introducteur de cathéter (12), de préférence dans lequel ledit premier élément d'imagerie de système électroanatomique, ledit deuxième élément d'imagerie de système électroanatomique et ledit troisième élément d'imagerie de système électroanatomique sont connectés de manière opérationnelle à un système de navigation électroanatomique (100).

4. Dispositif médical (10) selon la revendication 1, comprenant en outre une couche (40) de matériau thermorétractable adjacente à ladite couche externe (26) de telle sorte que ladite couche externe (26) se couple au revêtement interne (24) et à la couche (40) de matériau thermorétractable.

5. Dispositif médical (10) selon la revendication 1, dans lequel au moins une partie d'une extrémité distale de ladite couche externe (26) comprend au moins un parmi un matériau polymère transparent et un matériau polymère translucide, de préférence dans lequel une extrémité distale de ladite pluralité de tubes extrudés (38, 38₁-38₈) est adjacente à ladite partie de ladite couche externe (26) comprenant au moins un parmi un matériau polymère transparent et un matériau polymère translucide.

6. Dispositif médical (10) selon la revendication 1, dans lequel ledit élément d'imagerie de système électroanatomique (14) comprend au moins un parmi : un élément d'électrode de mesure d'impédance, un élément capteur de champ magnétique, un élément de système de télémétrie acoustique, un élément de bobine conductrice, un élément d'imagerie par tomodensimétrie, et un élément d'imagerie par résonance magnétique.

7. Dispositif médical (10) selon la revendication 1, dans lequel ledit élément d'imagerie de système électroanatomique (14) comprend un parmi : une électrode de stimulation, une électrode électrophysiologique, une électrode de positionnement, une électrode de cartographie, une électrode annulaire, une électrode solide et une électrode de pointe.

8. Système (98) comprenant :
un introducteur de cathéter (12) selon la revendication 1, et
un système de navigation électroanatomique (100) adapté pour recevoir des signaux dudit élément d'imagerie de système électroanatomique (14).

9. Système (98) selon la revendication 8, dans lequel ledit système de navigation électroanatomique (100) comprend des moyens pour effectuer au moins une parmi la détermination d'une position dudit élément d'imagerie de système électroanatomique (14) et la surveillance d'un signal électrophysiologique.

10. Système (98) selon la revendication 9, dans lequel lesdits moyens de détermination d'une position comprennent au moins un parmi : une électrode de mesure d'impédance, un élément de capteur de champ magnétique, un élément de système de télémétrie acoustique, un élément de bobine conductrice, un élément d'imagerie par tomodensitométrie, et un élément d'imagerie par résonance magnétique.

11. Système (98) selon la revendication 8, dans lequel ledit système de navigation électroanatomique (100) comprend un écran d'affichage (104), dans lequel ledit écran d'affichage (104) est adapté pour afficher une représentation visuelle d'un emplacement tridimensionnel d'un ou plusieurs cathéters.

12. Système (98) selon la revendication 9, comprenant en outre un second dispositif médical capable de délivrer ou de recevoir de l'énergie.
